# EUROPÄISCHE PATENTANMELDUNG

(11) **EP 2 292 299 A1**
(43) Veröffentlichungstag der Anmeldung: **09.03.2011**
(21) Anmeldenummer: 10172228.8
(22) Anmeldetag: 06.08.2010
(51) Int. Cl.: A61N 5/10

(54) **Strahlentherapievorrichtung zur Überwachung einer Bestrahlung**

(30) Priorität: 07.09.2009 DE 102009040389
(71) Anmelder: Siemens Aktiengesellschaft, 80333 München (DE)
(72) Erfinder: Rietzel, Eike, 64331, Weiterstadt (DE)

(57) **Zusammenfassung**

Die Erfindung betrifft eine Strahlentherapievorrichtung mit einer Quelle zur Bereitstellung eines Therapiestrahls, mit einer Bildgebungsvorrichtung, welche eine Röntgenstrahlenquelle und einen Röntgenstrahlendetektor umfasst, wobei die Röntgenstrahlenquelle und der Röntgenstrahlendetektor derart anordbar sind, dass von der Röntgenstrahlenquelle diagnostische Röntgenstrahlung durch ein zu bestrahlendes Zielobjekt auf den Röntgenstrahlendetektor gerichtet werden kann, und mit einer Steuerungsvorrichtung für die Bildgebungsvorrichtung, die ausgebildet ist, den Röntgenstrahlendetektor während einer Bestrahlung des Zielobjekts mit dem Therapiestrahl auszulesen, um Photonen, welche innerhalb des Zielobjekts aufgrund der Wechselwirkung des Therapiestrahls mit dem Zielobjekt entstehen, aufzuzeichnen. Weiterhin betrifft die Erfindung ein Verfahren bei dem das Zielobjekts mit einem Therapiestrahl bestrahlt wird und Photonen im Zielobjekt aufgrund der Wechselwirkung des Therapiestrahls mit dem Zielobjekt erzeugt werden. Die Bildgebungsvorrichtung wird verwendet um die Photonen aufzuzeichnen, welche anschließend ausgewertet werden.

## Beschreibung

Die Erfindung betrifft eine Strahlentherapievorrichtung mit einer Bildgebungsvorrichtung, mit der Bilder von dem zu bestrahlenden Objekt angefertigt werden können, sowie ein Verfahren zur Überwachung einer Bestrahlung eines Zielobjekts, bei welchem eine derartige Bildgebungsvorrichtung eingesetzt wird.

Eine Sonderform der Strahlentherapie ist die Partikeltherapie, die in den letzten Jahren zunehmend an Bedeutung gewinnt. Mit der Partikeltherapie können Verfahren zur Behandlung von Gewebe, insbesondere von Tumorerkrankungen, durchgeführt werden. Bestrahlungsverfahren, wie sie in der Partikeltherapie eingesetzt werden, finden jedoch auch in nichttherapeutischen Gebieten Anwendung. Hierzu gehören beispielsweise Forschungsarbeiten - etwa zur Produktentwicklung - im Rahmen der Partikeltherapie, die an nicht-lebenden Phantomen oder Körpern durchgeführt werden, Bestrahlungen von Materialien, etc.

Hierbei werden geladene Partikel wie z.B. Protonen, Kohlenstoffionen oder andere geladene Teilchen auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit einem Zielvolumen mit dem Partikelstrahl bestrahlt.

Durch die Wechselwirkung des Partikelstrahls mit dem Material des zu bestrahlenden Zielobjekts entstehen instabile Kerne, sowohl bei den Partikeln des Partikelstrahls als auch im Material des Zielobjekts. Es sind PET-Systeme (PET für Positronen-Emissions-Tomographie) bekannt, mit denen die Zerfälle dieser instabilen Kerne detektiert werden können.

Es ist die Aufgabe der Erfindung, eine Strahlentherapievorrichtung bereitzustellen, welche eine einfache und kostengünstige Überwachung der Zerfälle instabiler Kerne ermöglicht. Weiterhin ist die Aufgabe der Erfindung, ein entsprechendes Verfahren anzugeben.

Die Aufgabe der Erfindung wird gelöst durch die Gegenstände der unabhängigen Ansprüche. Vorteilhafte Weiterbildungen der Erfindung finden sich in den Merkmalen der abhängigen Ansprüche.

Eine erfindungsgemäße Strahlentherapievorrichtung weist auf:
- eine Quelle zur Bereitstellung eines Therapiestrahls, der von der Quelle auf ein zu bestrahlendes Zielobjekt richtbar ist,
- eine Bildgebungsvorrichtung, umfassend eine Röntgenstrahlenquelle und einen Röntgenstrahlendetektor, wobei die Röntgenstrahlenquelle und der Röntgenstrahlendetektor derart anordbar sind, dass von der Röntgenstrahlenquelle diagnostische Röntgenstrahlung durch das zu bestrahlende Zielobjekt auf den Röntgenstrahlendetektor gerichtet werden kann, und
- eine Steuerungsvorrichtung für die Bildgebungsvorrichtung, die derart ausgebildet ist, den Röntgenstrahlendetektor während einer Bestrahlung des Zielobjekts mit dem Therapiestrahl auszulesen, wodurch eine Aufzeichnung von Photonen, welche innerhalb des Zielobjekts aufgrund der Wechselwirkung des Therapiestrahls mit dem Zielobjekt entstehen, durchführbar ist.

Die Erfindung beruht auf der Erkenntnis, dass bei Strahlentherapievorrichtungen ein Patient oder ein anderes zu bestrahlendes Zielobjekt räumlich genau in Bezug auf den Therapiestrahl positioniert werden muss, um die gewünschte Bestrahlung zu gewährleisten. Hierfür wird üblicherweise eine Bildgebungsvorrichtung eingesetzt, welche es erlaubt, die interne Struktur des zu bestrahlenden Zielvolumens im Zielobjekt abzubilden. Es ist damit z.B. unmittelbar vor Beginn einer Bestrahlung möglich, die Lage des Zielvolumens zu ermitteln und gegebenenfalls entsprechend zu korrigieren. Die Bildgebungsvorrichtung kann auch während der Bestrahlung eingesetzt werden, um die Lage des Zielvolumens zu detektieren und zu überwachen, beispielsweise in einem fluoroskopieartigen Betrieb.

Es wurde allerdings erkannt, dass eine bereits vorhandene Bildgebungsvorrichtung auch zu einem anderen Zweck eingesetzt werden kann, nämlich zu dem Zweck, die in dem Zielvolumen aufgrund der Wechselwirkung des Therapiestrahl mit der Materie des Zielvolumens entstehenden radioaktiven Zerfälle über die daraus emittierten Photonen aufzuzeichnen. Da die in dem Zielvolumen auf diese Weise erzeugten Photonen als Maß für die im Zielvolumen deponierte Dosis gelten, kann auf so eine Überwachung der mit dem Therapiestrahl durchgeführten Dosisdeposition durchgeführt werden. Zusätzliche Geräte, wie ein zusätzliches PET-System oder ein SPECT-System, sind nicht notwendig, wodurch sich Kosten und Platzbedarf reduzieren. Insbesondere werden die auf diese Weise aufgezeichneten Photonen ausgewertet und können zur Steuerung der Strahlentherapievorrichtung verwendet werden, z.B. noch während einer Bestrahlungssitzung oder für nachfolgende Bestrahlungssitzungen.

In einer vorteilhaften Ausführungsform weist die Strahlentherapievorrichtung weiterhin eine Auswertungsvorrichtung auf, z.B. eine Rechnereinheit, die ausgebildet ist, eine durch den Therapiestrahl im Zielobjekt deponierte Dosisverteilung zumindest teilweise aus den mit dem Röntgenstrahlendetektor aufgezeichneten Photonen zu rekonstruieren. Auf diese Weise kann die deponierte Dosisverteilung bereits online während einer Bestrahlung oder aber nach einer Bestrahlung zumindest teilweise ermittelt werden, so dass der Bestrahlungserfolg überwacht werden kann. Wenn der Röntgenstrahlendetektor z.B. um das Zielobjekt bewegt wird, kann aus den aufgezeichneten Photonen eine sogar eine Dosisverteilung in drei Dimensionen ermittelt werden. Doch auch bei einem stationären Detektor, der Daten nur aus einer bestimmten Richtung aufzeichnet, können z.B. Rückschlüsse auf eine Reichweite des Therapiestrahls und/oder auf eine laterale Ausdehnung der deponierten Dosis gezogen werden.

In einer vorteilhaften Ausführungsform ist die Steuerungsvorrichtung derart ausgebildet, den Röntgenstrahlendetektor auszulesen ohne dabei gleichzeitig die Röntgenstrahlenquelle zu aktivieren. Die Bildgebungsvorrichtung kann somit in zwei unterschiedlichen Arten betrieben werden. In der ersten Betriebsart wird die Bildgebungsvorrichtung in einer Weise betrieben, bei der der Röntgenstrahlendetektor mit der Röntgenstrahlenquelle zusammenwirkt und ausgelesen wird, während die Röntgenstrahlenquelle aktiviert wird. In der zweiten Betriebsart hingegen wird der Röntgenstrahlendetektor alleine zur Detektion der im Zielobjekt entstandenen Photonen verwendet.

In einer vorteilhaften Ausführungsform ist die Bildgebungsvorrichtung an verschiedenen Positionen relativ zum Zielobjekt anordbar. Dies erlaubt es, die im die im Zielobjekt entstandenen Photonen aus verschiedenen Blickwinkeln aufzuzeichnen oder eine möglichst geeignete Lage des Röntgenstrahlendetektors relativ zum Zielobjekt und zum Strahlaustritt zu wählen, wodurch der Informationsgehalt der Aufzeichnung verbessert werden kann. Beispielsweise kann dies bei einer Rekonstruktion der deponierten Dosisverteilung hilfreich sein. Die Bildgebungsvorrichtung kann dazu beispielsweise an einer Positioniervorrichtung angeordnet sein, z.B. an einem Roboterarm, mit der sie an die verschiedenen Positionen bewegt werden kann. Hierzu kann beispielsweise die Bildgebungsvorrichtung derart angesteuert werden, dass die Position der Bildgebungsvorrichtung zur Aufzeichnung der durch den Therapiestrahl erzeugten Photonen während einer Bestrahlung des Zielvolumens geändert werden kann, um z.B. eine dreidimensionale Information über die deponierte Dosisverteilung zu erhalten. In einer vorteilhaften Ausgestaltung kann die Strahlentherapievorrichtung zumindest eine weitere Bildgebungsvorrichtung aufweisen, welche eine weitere Röntgenstrahlenquelle und einen weiteren Röntgenstrahlendetektor umfasst. Auch die weitere Bildgebungsvorrichtung kann wie die erste Bildgebungsvorrichtung ausgebildet sein und eingesetzt werden. Es ist aber auch möglich, die erste Bildgebungsvorrichtung in der ersten Betriebsart zu betreiben und die zweite Bildgebungsvorrichtung in der zweiten Betriebsart, sodass gleichzeitig eine Überwachung der Dosisdeposition und der Lage des Zielvolumens im Zielobjekt durchgeführt werden kann.

In einer vorteilhaften Ausführungsvariante weist der Röntgenstrahlendetektor eine Anpassvorrichtung zur Modifikation der auf den Röntgenstrahlendetektor treffenden Photonen auf, welche reversibel vor den Röntgenstrahlendetektor und insbesondere automatisch gesteuert platziert werden kann. Beispielsweise kann dann der Röntgenstrahlendetektor in einem ersten, herkömmlichen Bildgebungsmodus ohne die Anpassvorrichtung betrieben werden, während die Anpassvorrichtung im zweiten Betriebsmodus dafür sorgt, dass die im Zielobjekt durch Wechselwirkung mit dem Therapiestrahl entstandenen Photonen gefiltert und/oder modifiziert werden, bevor sie auf den Röntgenstrahlendetektor treffen.

Das erfindungsgemäße Verfahren weist folgende Schritte auf:
- Bestrahlen des Zielobjekts mit einem Therapiestrahl und erzeugen von Photonen im Zielobjekt aufgrund der Wechselwirkung des Therapiestrahls mit dem Zielobjekt
- Verwenden einer Bildgebungsvorrichtung, welche eine Röntgenstrahlenquelle und einen Röntgenstrahlendetektor zur Durchleuchtung des Zielobjekts mit einem Röntgenstrahl umfasst, zur Aufzeichnen der Photonen,
- Auswerten der aufgezeichneten Photonen.

Das Verfahren eignet sich sowohl im Rahmen einer Bestrahlung eines Patienten als auch im Rahmen einer Bestrahlung eines nicht-menschlichen oder nicht-tierischen Körpers, beispielsweise bei der Bestrahlung eines Phantoms, z.B. zu Test-, Forschungs- und/oder Kalibrierungszwecken.

In einer Ausführungsvariante werden die Photonen mit dem Röntgenstrahlendetektor aufgezeichnet, und zwar zu einem Zeitpunkt, an dem die Röntgenstrahlenquelle nicht aktiviert ist.

In vorteilhafter Weise werden die aufgezeichneten Photonen dadurch ausgewertet, dass aus den aufgezeichneten Photonen zumindest teilweise eine durch den Therapiestrahl im Zielobjekt deponierte Dosisverteilung rekonstruiert wird.

Der Röntgenstrahlendetektor kann während der Aufzeichnung der Photonen an verschiedene Position bewegt werden.

Zur Aufzeichnung der Photonen kann eine Anpassvorrichtung zur Modifikation der auf den Röntgenstrahlendetektor treffenden vor den Röntgenstrahlendetektor angeordnet werden, welche reversibel vor den Röntgenstrahlendetektor und insbesondere automatisch gesteuert platzierbar ist.

Der Röntgenstrahlendetektor kann also in einem Bildgebungsmodus betrieben werden, in welchem der Röntgenstrahlendetektor mit der Röntgenstrahlenquelle zusammenwirkt zur Aufzeichnung von Bilddaten eines zu bestrahlenden Objektes, und in einem Therapieüberwachungsmodus, in welchem der Röntgenstrahlendetektor ohne Zusammenwirken mit der Röntgenquelle die im Körper durch Wechselwirkung mit dem Therapiestrahl entstehenden Photonen aufzeichnet.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein. Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen dargestellten Aufbau einer Parti- keltherapieanlage;
- Fig. 2: eine schematische Ausführungsform der Strahlenthera- pievorrichtung mit einer Bildgebungsvorrichtung in ei- nem Bestrahlungsraum, welche im Bildgebungsmodus be- trieben wird,
- Fig. 3: eine Darstellung derselben Ausführungsform mit der Bildgebungsvorrichtung im Therapieüberwachungsmodus,
- Fig. 4: eine schematische Ausführungsform einer weiteren ähn- lichen Strahlentherapievorrichtung mit zwei Bildge- bungsvorrichtungen in einem Bestrahlungsraum, und
- Fig. 5: ein Diagramm mit Verfahrensschritten, welche bei der Durchführung einer Ausführungsform des Verfahrens aus- geführt werden.

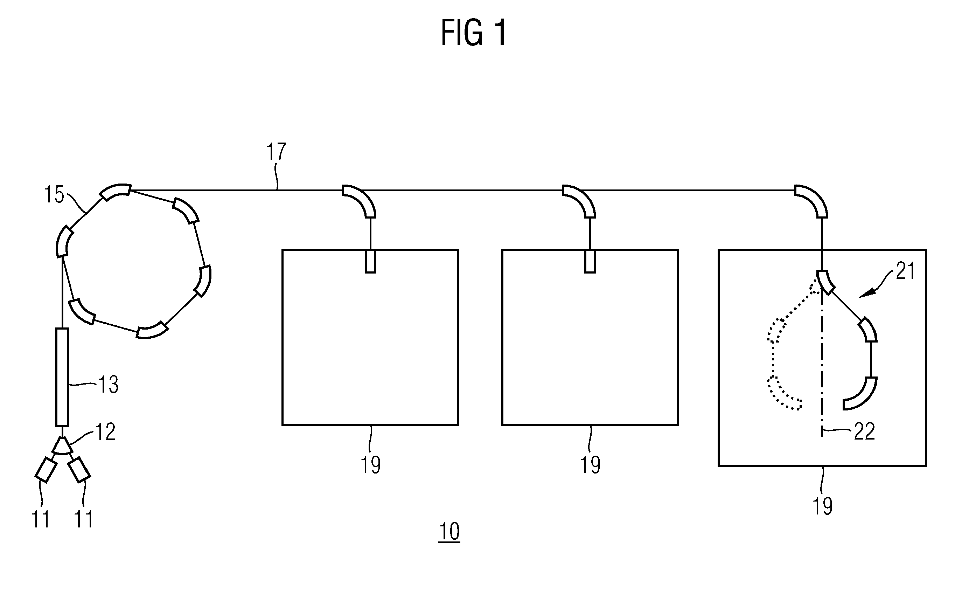

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Heliumionen, Kohlenstoffionen oder andere Teilchenarten wie Pionen eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der hier geschilderte Aufbau entspricht einer bekannten Partikeltherapieanlage 10. Einer derartige Partikeltherapieanlage 10 kann weitergebildet werden, sodass Ausführungsformen der vorliegenden Erfindung eingesetzt werden können.

Ausführungsformen der Erfindung können aber auch in anderen Partikeltherapieanlagen als in der hier gezeigten eingesetzt werden. Es ist aber auch denkbar, mit Röntgenstrahlen arbeitende Strahlentherapieanlagen zu verwenden, solange die Röntgenstrahlen derart beschaffen sind, dass durch Wechselwirkung im Zielobjekt weitere Photonen entstehen.

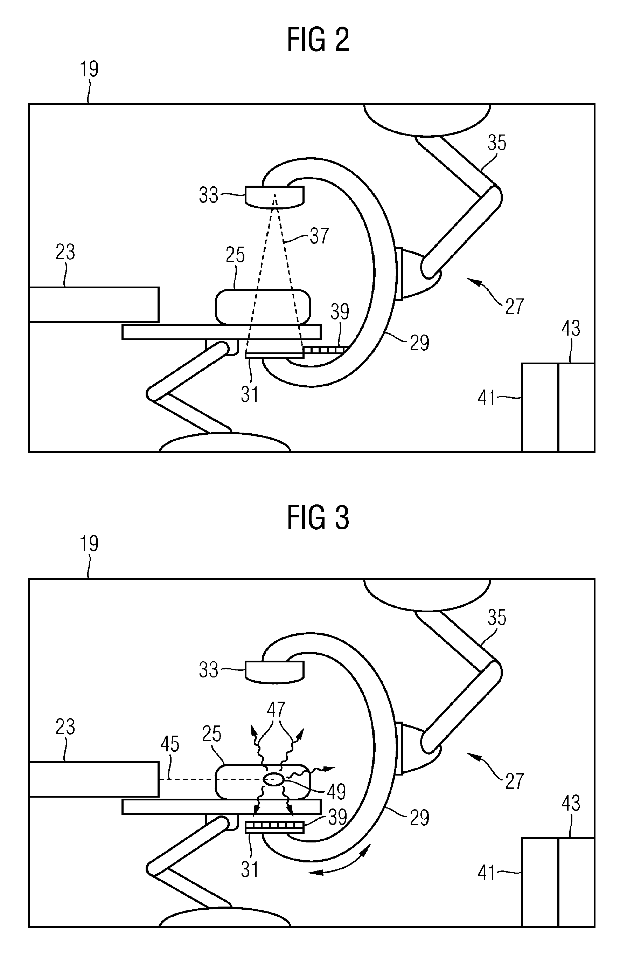

Ausführungsformen der Erfindung werden anhand der folgenden Figuren 2 bis 4 näher erläutert.

Fig. 2 zeigt schematisch den Aufbau eines Bestrahlungsraums 19, in welchem ein Partikelstrahl aus einer sogenannten Nozzle 23 austreten und auf ein zu bestrahlendes Zielobjekt 25 treffen kann.

Weiterhin ist in dem Raum eine Bildgebungsvorrichtung 27 angeordnet, hier dargestellt als ein C-Bogen 29 mit einem Röntgenstrahlendetektor 31 und einer Röntgenstrahlenquelle 33, welche über einen deckengehängten Roboterarm 35 bewegt werden können.

In Fig. 2 ist die Bildgebungsvorrichtung 27 in dem Bildgebungsmodus zu sehen. Das bedeutet, dass die Röntgenstrahlenquelle 33 aktiviert wird und dass hiermit ein Bild vom Inneren des zu bestrahlenden Zielobjekts 25 aufgezeichnet werden kann. Dies kann z.B. im Vorfeld einer Bestrahlungssitzung zur genauen Positionierung des Zielobjekts 25 erfolgen oder auch während der Bestrahlung zur Aufzeichnung und Überwachung der aktuellen Position des Zielobjekts 25. In Fig. 2 ist dies durch die angedeutete diagnostische Röntgenstrahlung 37 symbolisiert, die von der Röntgenstrahlenquelle 33 ausgeht und auf den Röntgendetektor 31 trifft. Eine Anpassvorrichtung 39, welche vor den Röntgenstrahlendetektor 31 gesteuert verfahren werden kann, befindet sich in einer Position, in der sie nicht im Strahlengang der Röntgenstrahlen 37 liegt.

Weiterhin ist in Fig. 2 eine Steuerungsvorrichtung 41 zur Steuerung der Bildgebungsvorrichtung 27 symbolisch dargestellt, mit der die Komponenten der Bildgebungsvorrichtung 27 positioniert und aktiviert werden können, sowie eine Auswertungsvorrichtung 43, mit der die von der Bildgebungsvorrichtung 27 erzeugten Messdaten ausgewertet und weiterverarbeitet werden können.

In Fig. 3 ist die Bildgebungsvorrichtung 27 in einem Therapieüberwachungsmodus dargestellt. Der Partikelstrahl 45 ist aktiviert, und durch Wechselwirkung mit dem Partikelstrahl 45 entstehen radioaktive Isotope im Inneren des Zielobjekts 25, durch deren Zerfall Photonen 47 generiert und aus dem Zielobjekt 25 emittiert werden. Die Steuerungsvorrichtung 41 steuert die Bildgebungsvorrichtung 27 dahingehend, dass der Röntgenstrahlendetektor 31 ohne Aktivierung der Röntgenstrahlenquelle 33 ausgelesen wird. Auf diese Weise lassen sich Informationen über die im Zielobjekt 25 entstandenen Photonen 47 ermitteln, insbesondere hinsichtlich ihrer räumlichen Verteilung und/oder Intensität. Die Auswertungsvorrichtung 41 kann beispielsweise aus dieser Information zumindest teilweise die deponierte Dosisverteilung 49 im Zielobjekt 25 rekonstruieren.

Im Therapiemodus ist die Anpassvorrichtung 39 vor dem Röntgenstrahlendetektor 31 angeordnet. Als Anpassvorrichtung 39 kann beispielsweise ein Streugitter eingesetzt werden oder aber auch eine Absorberplatte, sodass sich diejenigen Photonen verbessert filtern lassen, die nicht auf eine Wechselwirkung des Therapiestrahls 45 mit dem Zielobjekt 25 zurückzuführen sind oder die aus einer "falschen" Richtung auf den Röntgenstrahlendetektor 31 treffen. Der Therapiemodus gleicht somit einem SPECT-Aufnahmemodus (SPECT für engl.: "Single Photon Emission Computed Tomography").

Durch Bewegung der Bildgebungsvorrichtung 27 während der Bestrahlung und/oder unmittelbar nach der Bestrahlung lässt sich eine Verbesserung der Rekonstruktion der aufgezeichneten Photonen erreichen, da nun aus unterschiedlichen Richtungen Photonen 47 aufgezeichnet werden können. Die Bewegung ist durch den Doppelpfeil symbolisiert.

Die Aufzeichnung der im Zielobjekt entstehenden Photonen 47 ermöglicht beispielsweise einer nachträglichen Auswertung der deponierten Dosis 49, kann aber auch bereits online während einer Bestrahlung verwendet werden, beispielsweise um eine Bestrahlung abzubrechen, wenn eine Dosisdeposition außerhalb eines geplanten Bereichs festgestellt wird.

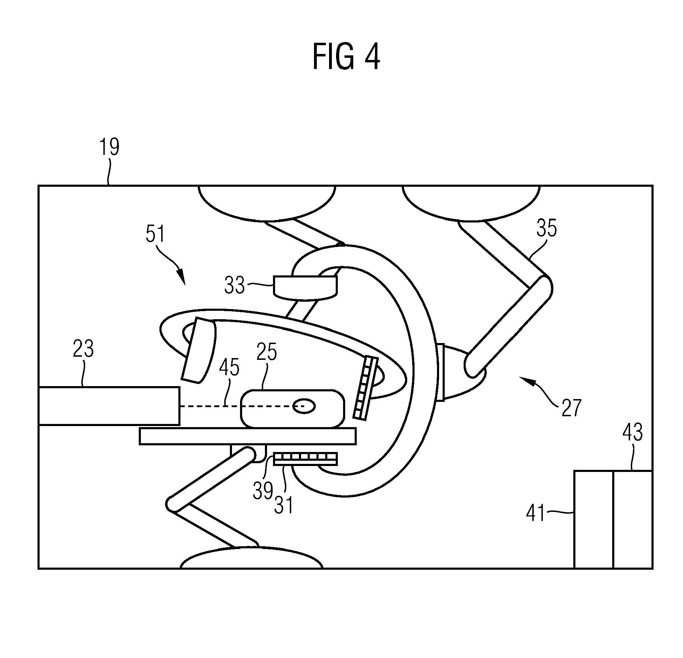

Fig. 4 zeigt eine Ausführungsvariante mit einer ersten Bildgebungsvorrichtung 27 und mit einer zweiten, analog aufgebauten Bildgebungsvorrichtung 51. Beide Bildgebungsvorrichtungen 27, 51 können auf die anhand von Fig. 2 und Fig. 3 beschriebene Weise verwendet und betrieben werden.

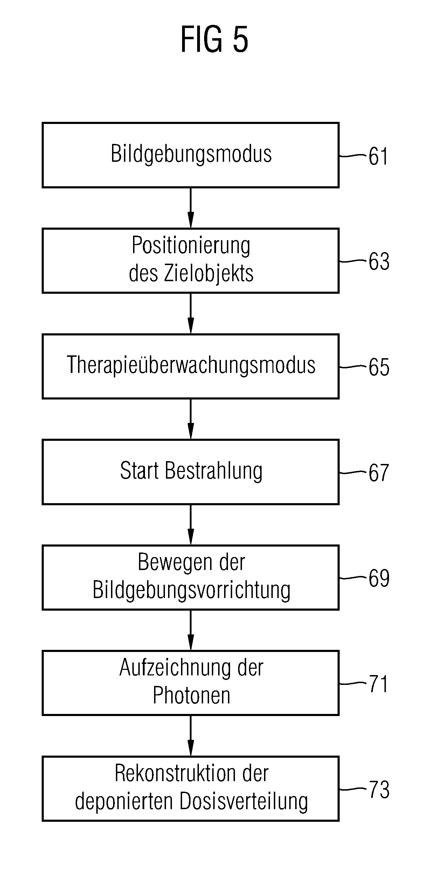

Fig. 5 zeigt einen schematischen Ablauf von Verfahrensschritten, die bei einer Ausführungsform der Erfindung eingesetzt werden können.

Zu Beginn wird das zu bestrahlende Zielobjekt in einem Bestrahlungsraum zu Bestrahlung positioniert, wobei der Bildgebungsmodus der Bildgebungsvorrichtung aktiviert wird (Schritt 61). Die Bildgebungsvorrichtung wird verwendet, um ein Bild vom Inneren des Zielobjekts anzufertigen, das beim Auffinden der korrekten Position und bei der Positionierung des Zielobjekts relativ zum Therapiestrahl verwendet wird (Schritt 63).

Anschließend wird die Bildgebungsvorrichtung von dem Bildgebungsmodus in den Therapieüberwachungsmodus umgeschaltet (Schritt 65). Die Bestrahlung wird gestartet (Schritt 67). Während der Bestrahlung kann die Bildgebungsvorrichtung an verschiedene Positionen bewegt werden (Schritt 69), um aus mehr als einer Richtung Photonen aufzeichnen zu können. Anschließend werden während der Bestrahlung und unmittelbar nach der Bestrahlung Photonen, die durch Wechselwirkung des Therapiestrahls mit dem Zielvolumen im Zielvolumen entstehen mit dem Röntgenstrahldetektor aufgezeichnet (Schritt 71).

Die aufgezeichneten Photonen können dazu verwendet werden, um die Bestrahlungsanlage zu steuern. Die aufgezeichneten Photonen können aber auch dazu dienen, die durch den Therapiestrahl deponierte Dosisverteilung zumindest teilweise zu rekonstruieren (Schritt 73).

### Bezugszeichenliste

- 10: Partikeltherapieanlage
- 11: Ionenquelle
- 12: Schaltmagnet
- 13: Vorbeschleuniger
- 15: Beschleuniger
- 17: Hochenergiestrahl-Transportsystem
- 19: Bestrahlungsraum
- 21: Gantry
- 22: Achse

- 23: Nozzle
- 25: Zielobjekt
- 27: Bildgebungsvorrichtung
- 29: C-Bogen
- 31: Röntgenstrahlendetektor
- 33: Röntgenstrahlenquelle
- 35: Roboterarm
- 37: diagnostische Röntgenstrahlung
- 39: Anpassvorrichtung
- 41: Steuerungsvorrichtung
- 43: Auswertungsvorrichtung

- 45: Partikelstrahl
- 47: Photonen
- 49: Dosisverteilung
- 51: zweite Bildgebungsvorrichtung

- 61: Schritt 61
- 63: Schritt 63
- 65: Schritt 65
- 67: Schritt 67
- 69: Schritt 69
- 71: Schritt 71
- 73: Schritt 73

## Patentansprüche

1. Strahlentherapievorrichtung, aufweisend:
- eine Quelle (11, 12, 13, 15, 17) zur Bereitstellung eines Therapiestrahls (45), der von der Quelle (11, 12, 13, 15, 17) auf ein zu bestrahlendes Zielobjekt (25) richtbar ist,
- eine Bildgebungsvorrichtung (27), umfassend eine Röntgenstrahlenquelle (33) und einen Röntgenstrahlendetektor (31), wobei die Röntgenstrahlenquelle (33) und der Röntgenstrahlendetektor (31) derart anordbar sind, dass von der Röntgenstrahlenquelle (33) diagnostische Röntgenstrahlung (37) durch das zu bestrahlende Zielobjekt (25) auf den Röntgenstrahlendetektor (31) gerichtet werden kann,
- eine Steuerungsvorrichtung (41) für die Bildgebungsvorrichtung (27), die derart ausgebildet ist, den Röntgenstrahlendetektor (33) während einer Bestrahlung des Zielobjekts (25) mit dem Therapiestrahl (45) auszulesen, um eine Aufzeichnung von Photonen (47), welche innerhalb des Zielobjekts (25) aufgrund der Wechselwirkung des Therapiestrahls (45) mit dem Zielobjekt (25) entstehen, durchzuführen.

2. Strahlentherapievorrichtung nach Anspruch 1, weiterhin aufweisend:
- eine Auswertungsvorrichtung (43), welche ausgebildet ist, eine durch den Therapiestrahl (45) im Zielobjekt (25) deponierte Dosisverteilung (49) zumindest teilweise aus den mit dem Röntgenstrahlendetektor (31) aufgezeichneten Photonen zu rekonstruieren.

3. Strahlentherapievorrichtung nach Anspruch 1 oder 2, wobei die Steuerungsvorrichtung (41) derart ausgebildet ist, den Röntgenstrahlendetektor (31) auszulesen ohne dabei gleichzeitig die Röntgenstrahlenquelle (33) zu aktivieren.

4. Strahlentherapievorrichtung nach einem der vorstehenden Ansprüche, bei welcher die Bildgebungsvorrichtung (27) an verschiedenen Positionen relativ zum Zielobjekt (25) positionierbar ist.

5. Strahlentherapievorrichtung nach Anspruch 4, bei welcher die Bildgebungsvorrichtung (27) mithilfe einer Positioniervorrichtung, insbesondere mithilfe eines Roboterarms (35), positionierbar ist.

6. Strahlentherapievorrichtung nach einem der vorstehenden Ansprüche, bei welcher die Bildgebungsvorrichtung (27) derart ansteuerbar ist, dass die Position des Röntgenstrahlendetektors (31) während der Aufzeichnung der Photonen (47) während einer Bestrahlung des Zielvolumens (25) änderbar ist.

7. Strahlentherapievorrichtung nach einem der vorstehenden Ansprüche, bei welcher zumindest eine weitere Bildgebungsvorrichtung (51), umfassend eine weitere Röntgenstrahlenquelle und einen weiteren Röntgenstrahlendetektor, vorgesehen ist.

8. Strahlentherapievorrichtung nach einem der vorstehenden Ansprüche, bei welcher der Röntgenstrahlendetektor (31) eine Anpassvorrichtung (39) zur Modifikation der auf den Röntgenstrahlendetektor (31) treffenden Photonen aufweist, welche reversibel und insbesondere automatisch gesteuert vor den Röntgenstrahlendetektor (31) anordbar ist.

9. Verfahren zur Überwachung einer Bestrahlung eines Zielobjekts (25), aufweisend folgende Schritte:
- Bestrahlen des Zielobjekts (25) mit einem Therapiestrahl (45) und erzeugen von Photonen (47) im Zielobjekt (45) aufgrund der Wechselwirkung des Therapiestrahls (45) mit dem Zielobjekt (25),
- Verwenden einer Bildgebungsvorrichtung (27), welche eine Röntgenstrahlenquelle (33) und einen Röntgenstrahlendetektor (31) zur Durchleuchtung des Zielobjekts (25) mit einem Röntgenstrahl (27) umfasst, zur Aufzeichnen der Photonen (47) mit dem Röntgenstrahlendetektor (31),
- Auswerten der aufgezeichneten Photonen (47).

10. Verfahren nach Anspruch 9, wobei die Photonen mit dem Röntgenstrahlendetektor (31) der Bildgebungsvorrichtung (27) aufgezeichnet werden ohne dass die Röntgenstrahlenquelle (33) aktiviert wird.

11. Verfahren nach Anspruch 9 oder 10, wobei die Auswertung dadurch stattfindet, dass aus den aufgezeichneten Photonen (47) zumindest teilweise eine durch den Therapiestrahl (45) im Zielobjekt (25) deponierte Dosisverteilung (49) rekonstruiert wird.

12. Verfahren nach einem der Ansprüche 9 bis 11, wobei der Röntgenstrahlendetektor (31) bei der Aufzeichnung der Photonen (47) an verschiedene Positionen bewegt wird.

13. Verfahren nach einem der Ansprüche 9 bis 12, wobei zur Aufzeichnung der Photonen (47) eine Anpassvorrichtung (39) zur Modifikation der auf den Röntgenstrahlendetektor (31) treffenden Photonen vor den Röntgenstrahlendetektor (31) angeordnet wird, wobei die Anpassvorrichtung (39) reversibel und insbesondere automatisch gesteuert angeordnet wird.

14. Verfahren nach Anspruch 13, wobei der Röntgenstrahlendetektor (31) in einem Bildgebungsmodus betreibbar ist, in welchem der Röntgenstrahlendetektor (31) mit der Röntgenstrahlenquelle (33) zur Aufzeichnung von Bilddaten des zu bestrahlenden Zielobjekts (25) zusammenwirkt, und in einem Therapieüberwachungsmodus, in welchem der Röntgenstrahlendetektor (31) ohne Zusammenwirkung mit der Röntgenstrahlenquelle (33) die im Zielobjekt (25) durch Wechselwirkung mit dem Therapiestrahl (45) entstehenden Photonen (47) aufzeichnet.
